Europäisches Patentamt

European Patent Office

Office européen des brevets

(11) Veröffentlichungsnummer: **0 123 256**
**B1**

(12) **EUROPÄISCHE PATENTSCHRIFT**

(45) Veröffentlichungstag der Patentschrift:
29.03.89

(21) Anmeldenummer: 84104322.7

(22) Anmeldetag: 17.04.84

(51) Int. Cl.⁴: **C 07 D 471/06** //
C09B5/62 ,(C07D471/06,
221:00, 221:00)

(54) Verfahren zur Herstellung von 3,4,9,10-Perylentetracarbonsäurediimid.

(30) Priorität: 21.04.83 DE 3314430
17.12.83 DE 3345810

(43) Veröffentlichungstag der Anmeldung:
31.10.84 Patentblatt 84/44

(45) Bekanntmachung des Hinweises auf die Patenterteilung:
29.03.89 Patentblatt 89/13

(84) Benannte Vertragsstaaten:
BE CH DE FR GB IT LI

(56) Entgegenhaltungen:
EP-A-0 054 806
FR-A-1 580 971

**Die Akte enthält technische Angaben, die nach
dem Eingang der Anmeldung eingereicht wurden
und die nicht in dieser Patentschrift enthalten sind.**

(73) Patentinhaber: **HOECHST AKTIENGESELLSCHAFT,
Postfach 80 03 20, D-6230 Frankfurt am Main 80
(DE)**

(72) Erfinder: **Spietschka, Ernst, Dr., Kirchweg 3,
D-6270 Idstein/Taunus (DE)**
Erfinder: **Urban, Manfred, Steigerwaldstrasse 2A,
D-6200 Wiesbaden (DE)**

EP 0 123 256 B1

## Beschreibung

Gegenstand der Erfindung ist ein Verfahren zur Herstellung von hochreinem 3,4,9,10-Perylentetracarbonsäurediimid in hoher Ausbeute.

3,4,9,10-Perylentetracarbonsäurediimid und Verfahren zu seiner Herstellung sind bekannt. So kann gemäß der Deutschen Patentschrift 386 057 das 3,4,9,10-Perylentetracarbonsäurediimid durch Umsetzung von 3,4,9,10-Perylentetracarbonsäuredianhydrid mit Ammoniak hergestellt werden. Das nach diesem Verfahren erhältliche Produkt ist zwar sehr rein, das Verfahren hat aber den Nachteil, daß es sehr aufwendig ist, weil das als Reaktionskomponente dienende 3,4,9,10-Perylentetracarbonsäuredianhydrid selbst aus dem 3,4,9,10-Perylentetracarbonsäurediimid durch Verseifen mit konzentrierter Schwefelsäure gewonnen wird. Damit scheidet dieses Verfahren als Lösung der technischen Aufgabe, 3,4,9,10-Perylentetracarbonsäurediimid aus großtechnisch leicht zugänglichen Naphthalinderivaten herzustellen, aus.

Nach der Deutschen Patentschrift 276 357 kann das 3,4,9,10-Perylentetracarbonsäurediimid durch Erhitzen einer Mischung aus 1,8-Naphthalindicarbonsäureimid Ätzkali und geringen Mengen Wasser auf Temperaturen von 280 - 300° C hergestellt werden. Angaben über Ausbeute und Reinheit des Reaktionsprodukts werden in der genannten Patentschrift aber nicht gemacht. Nach dem dort beschriebenen Verfahren wird das 3,4,9,10-Perylentetracarbonsäurediimid in unbefriedigender Reinheit und Ausbeute erhalten.

Gemäß Chemical Abstracts 1948, 5892 wird ein 3,4,9,10-Perylentetracarbonsäurediimid in einer Ausbeute von 75 - 81 % der Theorie durch Verschmelzen von 1,8-Naphthalindicarbonsäureimid mit Ätzalkali unter Zusatz von Wasser erhalten, das nach Spektraluntersuchungen nahezu rein sein soll. Bei der Nacharbeitung der Angaben in Chemical Abstracts 1948, 5892 in zehnfacher Ansatzgröße konnte die Behauptung der Entstehung einer Lösung des 3,4,9,10-Perylentetracarbonsäurediimids, aus der durch Ansäuern das genannte Diimid gewonnen wird, nicht bestätigt werden.

Bei Verwendung von Natriumhydroxid als Schmelzmittel wird ein rohes 3,4,9,10-Perylentetracarbonsäurediimid in einer Ausbeute von 46,1 % der Theorie erhalten. Bei der üblichen Reinheitsbestimmungsmethode wird auf der Stufe des reinen 3,4,9,10-Perylentetracarbonsäurediimids eine Ausbeute von nur 57,8 %, bezogen auf rohes 3,4,9,10-Perylentetracarbonsäurediimid, erreicht. Falle der Verwendung von Ätzkali liegt die Rohausbeute bei 81,2 % der Theorie und die Reinheitsbestimmung liefert 89,7 %.

In der Europäischen Patentanmeldung 0 054 806 wird ein Verfahren zur Herstellung von 3,4,9,10-Perylentetracarbonsäurediimid durch Verschmelzen von 1,8-Naphthalindicarbonsäureimid in einer Mischung aus Ätzkali, Ätznatron und Natriumazetat bei Temperaturen von 190 - 220° C und anschließende Oxidation, vorzugsweise in der Schmelze, beschrieben. Es werden Ausbeuten von bis zu 93,4 % angegeben und die Reinheit des erhältlichen Produkts wird als hoch bezeichnet. Eine Bestimmungsmethode wird jedoch nicht angegeben.

Gemäß Beispiel 2 von Fr-A-1 580 971 wird 1,8-Naphthalindicarbonsäureimid mit Ätzkali und Natriumacetat bei 230° bis 240° C erhitzt. Anschließend wird das Produkt in Wasser gelöst und in die Lösung Luft eingeleitet, wobei das rohe Perylen-3,4,9,10-tetracarbonsäurediimid gebildet wird, welches dann durch Vermahlen in einem inerten organischen Lösungsmittel bei einer Temperatur bis etwa 50° C in eine coloristisch wertvolle Pigmentform übergeführt wird.

Es wurde nun gefunden, daß man 3,4,9,10-Perylentetracarbonsäurediimid mit hohem Reinheitsgrad und in hoher Ausbeute durch Verschmelzen von 1,8-Naphthalindicarbonsäureimid mit Ätzkali und Natriumazetat zum Alkalimetallsalz der Leukoverbindung des 3,4,9,10-Perylentetracarbonsäurediimids, Verdünnen der Schmelze mit Wasser nach beendeter Reaktion, und Oxidation der Leukoform zum 3,4,9,10-Perylentetracarbonsäurediimid herstellen kann, indem man

a)   vor dem Eintragen des 1,8-Naphthalindicarbonsäureimids in die Ätzkali-Natriumacetat-Schmelze ein Vakuum von weniger als 133,3 mbar (100 Torr) anlegt, die Ätzkali-Natriumacetat-Mischung auf 200 - 300° C, vorzugsweise 250 - 300° C, erhitzt und diese hierbei soweit entwässert, daß die angefallene Schmelze bei 200 - 220° C unter technischen Bedingungen noch rührbar ist, anschließend das 1,8-Naphthalindicarbonsäureimid oder dessen Natrium- oder Kaliumsalz bei 200 - 220° C bei Normaldruck in die genannte Schmelze einträgt und diese unter Ausschluß von Luftsauerstoff auf 250 - 300° C bis zur vollständigen Bildung des Alkalimetallsalzes der Leukoverbindung erhitzt, oder

b)   nach dem Eintragen des 1,8-Naphthalindicarbonsäureimids oder dessen Natrium- oder Kaliumsalzes bei 200 - 220° C bei Normaldruck in die Ätzkali-Natriumacetat-Schmelze ein Vakuum von weniger als 133,3 mbar (100 Torr) anlegt, die Mischung auf 200 - 250° C erhitzt und diese hierbei soweit entwässert, daß die angefallene Schmelze bei 200 - 220° C unter technischen Bedingungen noch rührbar ist, und anschließend die Schmelze unter Ausschluß von Luftsauerstoff auf 250 - 300° C bis zur vollständigen Bildung des Alkalimetallsalzes der Leukoverbindung erhitzt, oder

c) in die Ätzkali-Natriumacetat-Schmelze das Natrium- oder Kaliumsalz des 1,8-Naphthalindicarbonsäureimids bei 200 - 220°C bei Normaldruck einträgt und die Schmelze unter Ausschluß von Luftsauerstoff auf 250 - 300°C bis zur vollständigen Bildung des Alkalimetallsalzes der Leukoverbindung erhitzt.

Im einzelnen ist es für die Verfahrensdurchführung zweckmäßig, auf 1 Gewichtsteil 1,8-Naphthalindicarbonsäureimid bzw. dessen Äquivalent an Natrium- oder Kaliumsalz 3 bis 10 Gewichtsteile, vorzugsweise 3 bis 6 Gewichtsteile Ätzkali und 0,1 bis 2 Gewichtsteile, vorzugsweise 0,3 bis 0,8 Gewichtsteile Natriumacetat einzusetzen.

Die Oxidation der erfindungsgemäß auf einem der vorstehend beschriebenen Wege a), b) oder c) intermediär angefallenen Leukoverbindung des 3,4,9,10-Perylentetracarbonsäurediimids erfolgt auf an sich bekannte Weise, beispielsweise durch Verblasen mit Luft [BIOS Final Report, 1484, Seite 21] oder mit Peroxodisulfaten, Nitraten, Chloraten, Addukten von Wasserstoffperoxid an Borate und Hypochlorit-Lösungen gemäß den Angaben der europäischen Patentanmeldung 0 054 806.

Was die beim erfindungsgemäßen Verfahren zur Anwendung gelangenden Verbindungen Ätzkali und Natriumacetat anbelangt, so wird das Natriumacetat in wasserfreier Form eingesetzt, während unter dem angewandten Ätzkali technisch verfügbares Ätzkali eines Wassergehalts von 10 - 15 % zu verstehen ist.

Unter dem erfindungsgemäß eingesetzten 1,8-Naphthalindicarbonsäureimid bzw. dessen Natrium- oder Kaliumsalz ist jeweils die wasserfreie Verbindung zu verstehen, wobei das Natrium- bzw. Kaliumsalz bevorzugt eingesetzt wird (Vermeidung der Wasserbildung in der Schmelze durch Umsetzung von KOH mit dem 1,8-Naphthalindicarbonsäureimid).

Es ist als überraschend zu erachten, daß das 3,4,9,10-Perylentetracarbonsäurediimid erfindungsgemäß, d. h. bei relativ hohen Reaktionstemperaturen und/oder langen Reaktionszeiten erhalten werden kann, nachdem in der europäischen Patentanmeldung 0 054 806 beschrieben wird, daß möglichst kurze Reaktionszeit und möglichst niedrige Reaktionstemperatur zu hoher Ausbeute und hoher Reinheit führen. Das nach dem Verfahren der genannten europäischen Patentanmeldung hergestellte 3,4,9,10-Perylentetracarbonsäurediimid ergab nach der Gehaltsbestimmung einen Reinheitsgehalt von 81 %, während die nach dem erfindungsgemäßen Verfahren hergestellten 3,4,9,10-Perylentetracarbonsäurediimide Reinheitsgehalte von 90 - 95 % aufweisen.

Die auf dem erfindungsgemäßen Weg erhaltenen 3,4,9,10-Perylentetracarbonsäurediimide eignen sich hervorragend für die Herstellung von 3,4,9,10-Perylentetracarbonsäurediimid in Pigmentform sowie für Alkylierungsprodukte und als Ausgangsprodukt zur Herstellung von 3,4,9,10-Perylentetracarbonsäuredianhydrid.

**Beispiel 1**

235 g Ätzkali (85 %-ig) und 22,5 g Natriumazetat (wasserfrei) werden in einem Edelstahlgefäß mit Rührwerk vorgelegt. Dann wird ein Vakuum 3,999 mbar (3 Torr) angelegt und 2 Stunden auf 300°C erhitzt, wobei 25 g Wasser entweichen. Anschließend läßt man auf 200°C abkühlen und trägt dann bei dieser Temperatur innerhalb 1 Stunde 50 g 1,8-Naphthalindicarbonsäureimid (98,1 %-ig) unter Überleiten von Stickstoff ein. Dann heizt man auf 300°C auf und rührt bei dieser Temperatur 6 Stunden nach. Nach Abkühlen auf 200°C läßt man, beginnend bei dieser Temperatur, 300 ml Wasser zutropfen, gießt dann die so verdünnte Schmelze auf 1500 ml Wasser, leitet 6 Stunden bei Raumtemperatur Luft bis zur vollständigen Oxidation der Leukoverbindung ein, saugt den Niederschlag ab, wäscht den Filterrückstand mit Wasser und trägt ihn dann in verdünnte Salzsäure ein. Nach einstündigem Nachrühren bei 75°C wird das Produkt abgesaugt, neutral gewaschen und bei 80°C getrocknet. Gewonnen werden 44,90 g 3,4,9,10-Perylentetracarbonsäurediimid roh.

Bestimmung des Reingehaltes an 3,4,9,10 Perylencarbonsäurediimid: 20 g des wie vorstehend beschrieben hergestellten rohen 3,4,9,10-Perylencarbonsäurediimids werden in 400 g konzentrierte Schwefelsäure eingetragen. Nach Erwärmen der Mischung auf 80°C und Lösen des genannten Rohprodukts läßt man innerhalb 1 Stunde 150 g Schwefelsäure (50 %-ig) zutropfen, wobei die Temperatur auf 100°C ansteigen darf. Anschließend läßt man auf 25°C abkühlen und saugt den Niederschlag ab, wäscht diesen mit 78 %-iger Schwefelsäure bis zum klaren Ablauf, wäscht anschließend neutral und trocknet schließlich bei 80°C. Man erhält 18,48 g reines 3,4,9,10-Perylencarbonsäurediimid. Das erhaltene 3,4,9,10-Perylentetracarbonsäurediimid roh ist damit 92,4 %-ig. Dies entspricht einer Reinausbeute von 85,5 % der Theorie, bezogen auf 100 %-iges 1,8-Naphthalindicarbonsäureimid.

**Beispiel 2**

235 g Ätzkali (90 %-ig) und 22,5 6 Natriumazetat (wasserfrei) werden in einem Edelstahlgefäß mit Rührwerk vorgelegt. Nach Aufheizen der Mischung auf 200°C werden innerhalb 1 Stunde 62,5 g des Kaliumsalzes des 1,8-Naphthalindicarbonsäureimids (entsprechend 50 g 1,8-Naphthalindicarbonsäureimid 100 %-ig) eingetragen unter Überleiten von Stickstoff. Nach Aufheizen auf 300°C und sechsstündigem Nachrühren bei dieser Temperatur läßt man auf 200°C abkühlen und dann, bei dieser Temperatur beginnend, langsam 300 ml Wasser zutropfen. Anschließend gießt man die verdünnte Schmelze auf 1500 ml Wasser, leitet 6 Stunden Luft bei Raumtemperatur bis zur vollständigen Oxidation der Leukoverbindung ein, saugt den Niederschlag ab, wäscht diesen mit Wasser, trägt den Filterrückstand in verdünnte Salzsäure ein, rührt 1 Stunde bei 75°C nach, saugt den Niederschlag ab, wäscht diesen neutral und trocknet schließlich bei 80°C.

Gewonnen werden 46,22 g 3,4,9,10-Perylentetracarbonsäurediimid (95,0 %-ig), was einer Reinausbeute von 88,7 % der Theorie, bezogen auf 100 %-iges 1,8-Naphthalintetracarbonsäureimid entspricht. (Die Bestimmung des Reingehalts an 3,4,9,10-Perylencarbonsäurediimid erfolgt nach der in Beispiel 1 angegebenen Methode).

**Beispiel 3**

155 g Ätzkali (90 %-ig) und 25 g Natriumacetat (wasserfrei) werden in einem Edelstahlgefäß mit Rührwerk vorgelegt. Nach dem Aufheizen der Mischung auf 200°C werden innerhalb 1 Stunde 60,6 g des Kaliumsalzes des 1,8-Naphthalindicarbonsäureimids (ensprechend 50 g 1,8-Naphthalindicarbonsäureimid 100 %-ig) bei der genannten Temperatur eingetragen unter Überleiten von Stickstoff. Dann wird ein Vakuum 52,32 - 66,65 mbar (40 - 50 Torr) angelegt und in 2 Stunden bei einer Temperatur bis 250°C entwässert, wobei 6,9 g Wasser entweichen. Anschließend wird unter Normaldruck, auf 260°C geheizt und 4 Stunden bei dieser Temperatur unter Überleiten von Stickstoff nachgerührt. Nach Abkühlen auf 200°C läßt man, bei dieser Temperatur beginnend, 300 ml Wasser zutropfen, gießt dann die verdünnte Schmelze auf 1500 ml Wasser, leitet 6 Stunden bei Raumtemperatur Luft bis zur vollständigen Oxidation der Leukoverbindung ein, saugt den Niederschlag ab, wäscht diesen mit Wasser und trägt ihn dann in verdünnte Salzsäure ein. Nach einstündigem Rühren bei 75°C wird das Produkt ab gesaugt, neutral gewaschen und bei 80°C getrocknet.

Gewonnen werden 45,5 g 3,4,9,10-Perylentetracarbonsäurediimid (91,0 %-ig), das eine Reinausbeute von 83,3 % der Theorie, bezogen auf 100 %-iges 1,8-Naphthalindicarbonsäureimid, entspricht. (Die Bestimmung des Reingehalts an 3,4,9,10-Perylentetracarbonsäurediimid erfolgt nach der in Beispiel 1 angegebenen Methode).

**Beispiel 4**

235 g Ätzkali (90 %-ig) und 22,5 g Natriumazetat (wasserfrei) werden in einem Edelstahlgefäß mit Rührwerk vorgelegt. Dann wird ein Vakuum (40 - 50 Torr) angelegt und 2 Stunden auf 300°C erhitzt, wobei 12,4 g Wasser entweichen. Anschließend läßt man auf 200°C abkühlen und trägt dann bei dieser Temperatur innerhalb 1 Stunde 62,5 g des Kaliumsalzes des 1,8-Naphthalindicarbonsäureimids (entsprechend 50 g 1,8-Naphthalindicarbonsäureimid (100 %-ig) unter Überleiten von Stickstoff ein. Darauf heizt man auf 300°C auf und rührt 6 Stunden bei dieser Temperatur nach. Nach Abkühlen auf 200°C läßt man, bei dieser Temperatur beginnend, 300 ml Wasser zutropfen, gießt dann die verdünnte Schmelze auf 1500 ml Wasser, leitet 6 Stunden bei Raumtemperatur Luft bis zur vollständigen Oxidation ein, saugt den Niederschlag ab, wäscht diesen mit Wasser und trägt ihn dann in verdünnte Salzsäure ein. Nach einstündigem Rühren bei 75°C wird das Produkt abgesaugt, neutral gewaschen und bei 80°C getrocknet.

Gewonnen werden 48,0 g 3,4,9,10-Perylentetracarbonsäurediimid (95 %-ig), was einer Reinausbeute von 94,1 % der Theorie, bezogen auf 100 %-iges 1,8-Naphthalindicarbonsäureimid, entspricht. (Die Bestimmung des Reingehalts an 3,4,9,10-Perylentetracarbonsäurediimid erfolgt nach der in Beispiel 1 angegebenen Methode).

**Beispiel 5**

235 g Ätzkali (85 %-ig) und 22,5 g Natriumazetat (wasserfrei) werden in einem Edelstahlgefäß mit Rührwerk vorgelegt. Dann wird ein Vakuum 3,999 mbar (3 Torr) angelegt und 2 Stunden auf 300°C erhitzt, wobei 24,8 g Wasser entweichen. Anschließend läßt man auf 200°C abkühlen und trägt dann bei dieser Temperatur innerhalb 1 Stunde 62,5 g des Kaliumsalzes des 1,8-Naphthalindicarbonsäureimids (entsprechend 50 g 1,8-Naphthalindicarbonsäureimid 100 %-ig) unter Überleiten von Stickstoff ein. Dann erhitzt man auf 250°C und rührt 6 Stunden bei dieser Temperatur nach. Nach Abkühlen auf 200°C läßt man, beginnend bei dieser Temperatur, 300 ml Wasser zutropfen, gießt die verdünnte Schmelze auf 1500 ml Wasser, trennt die entstandene Leukoverbindung durch Filtrieren von der Mutterlauge ab, rührt den Filterrückstand mit der fünffachen Menge Wasser an, leitet danach bis zur vollständigen Oxidation Luft ein, stellt mit verdünnter Salzsäure auf pH 3, saugt ab, wäscht neutral und trocknet bei 80°C.

Gewonnen werden 48,8 g 3,4,9,10-Perylentetracarbonsäurediimid (91,3 %-ig), was einer Reinausbeute von 90,0 % der Theorie, bezogen auf 100 %-iges 1,8-Naphthalindicarbonsäureimid, entspricht. (Die Bestimmung des Reingehalts an 3,4,9,10-Perylentetracarbonsäurediimid erfolgt nach der in Beispiel 1 angegebenen Methode).

**Beispiel 6**

235 g Ätzkali (85 %-ig) und 22,5 g Natriumazetat (wasserfrei) werden in einem Edelstahlgefäß mit Rührwerk vorgelegt. Dann wird ein Vakuum 3,999 mbar (3 Torr) angelegt und 2 Stunden auf 300° C erhitzt, wobei 24,9 g Wasser entweichen. Anschließend läßt man auf 200° C abkühlen und trägt dann bei dieser Temperatur innerhalb 1 Stunde 62,5 g des Kaliumsalzes des 1,8-Naphthalindicarbonsäureimids (entsprechend 50 g 1,8-Naphthalindicarbonsäureimid 100 %-ig) unter Überleiten von Stickstoff ein. Dann heizt man auf 280° C auf und rührt bei dieser Temperatur 2 Stunden nach. Nach Abkühlen auf 200° C läßt man, beginnend bei dieser Temperatur, 300 ml Wasser zutropfen, gießt die verdünnte Schmelze auf 1500 ml Wasser, leitet 6 Stunden bei Raumtemperatur Luft bis zur vollständigen Oxidation der Leukoverbindung ein, saugt den Niederschlag ab, wäscht den Filterrückstand mit Wasser und trägt ihn dann in verdünnte Salzsäure ein. Nach einstündigem Nachrühren bei 75° C wird das Produkt abgesaugt, neutral gewaschen und bei 80° C getrocknet.

Gewonnen werden 47,9 g 3,4,9,10-Perylentetracarbonsäurediimid (91,6 %-ig), was einer Reinausbeute von 88,7 % der Theorie, bezogen auf 100 %-iges 1,8-Naphthalindicarbonsäureimid, entspricht. (Die Bestimmung des Reingehalts an 3,4,9,10-Perylentetracarbonsäurediimid erfolgt nach der in Beispiel 1 beschriebenen Methode).

**Beispiel 7**

235 g Ätzkali (85 %-ig) und 22,5 g Natriumazetat (wasserfrei) werden in einem Edelstahlgefäß mit Rührwerk vorgelegt. Dann wird ein Vakuum 3,999 mbar (3 Torr) angelegt und 2 Stunden auf 300° C erhitzt, wobei 26,3 g Wasser entfernt werden. Anschließend läßt man auf 200° C abkühlen und trägt dann bei dieser Temperatur innerhalb 1 Stunde 61,7 g des Natriumsalzes des 1,8-Naphthalindicarbonsäureimids (entsprechend 50 g 1,8-Naphthalindicarbonsäureimid 100 %-ig), unter Überleiten von Stickstoff ein. Dann heizt man auf 300° C auf und rührt 6 Stunden bei dieser Temperatur nach. Nach Abkühlen auf 200° C läßt man, beginnend bei dieser Temperatur, 300 ml Wasser zutropfen, gießt die verdünnte Schmelze auf 1500 ml Wasser, leitet 6 Stunden bei Raumtemperatur Luft bis zur vollständigen Oxidation der Leukoverbindung ein, saugt den Niederschlag ab, wäscht den Filterrückstand mit Wasser und trägt ihn dann in verdünnte Salzsäure ein. Nach einstündigem Nachrühren bei 75° C wird das Produkt abgesaugt, neutral gewaschen und bei 80° C getrocknet.

Gewonnen werden 46,8 g 3,4,9,10-Perylentetracarbonsäurediimid (95,0 %-ig), was einer Reinausbeute von 89,8 % der Theorie, bezogen auf 100 %-iges Naphthalindicarbonsäureimid, entspricht. (Diese Bestimmung des Reingehalts an 3,4,9,10-Perylentetracarbonsäurediimid erfolgt nach der in Beispiel 1 beschriebenen Methode).

**Patentansprüche**

1. Verfahren zur Herstellung von 3,4,9,10-Perylentetracarbonsäurediimid mit hohem Reinheitsgrad und in hoher Ausbeute durch Verschmelzen von 1,8-Naphthalindicarbonsäureimid mit Ätzkali und Natriumazetat zum Alkalimetallsalz der Leukoverbindung des 3,4,9,10-Perylentetracarbonsäurediimids, Verdünnen der Schmelze mit Wasser nach beendeter Reaktion, und Oxidation der Leukoform zum 3,4,9,10-Perylentetracarbonsäurediimid, dadurch gekennzeichnet, daß man

a) vor dem Eintragen des 1,8-Naphthalindicarbonsäureimids in die Ätzkali-Natriumacetat-Schmelze ein Vakuum von weniger als 133,3 mbar (100 Torr) anlegt, die Ätzkali-Natriumacetat-Mischung auf 200 - 300° C, erhitzt und diese hierbei soweit entwässert, daß die angefallene Schmelze bei 200 - 220° C unter technischen Bedingungen noch rührbar ist, anschließend das 1,8-Naphthalindicarbonsäureimid oder dessen Natrium- oder Kaliumsalz bei 200 - 220° C bei Normaldruck in die genannte Schmelze einträgt und diese unter Ausschluß von Luftsauerstoff auf 250 - 300° C bis zur vollständigen Bildung des Alkalimetallsalzes der Leukoverbindung erhitzt, oder

b) nach dem Eintragen des 1,8-Naphthalindicarbonsäureimids oder dessen Natrium- oder Kaliumsalzes bei 200 - 220° C bei Normaldruck in die Ätzkali- Natriumacetat-Schmelze ein Vakuum von weniger als 133,3 mbar (100 Torr) anlegt, die Mischung auf 200 - 250° C erhitzt und diese hierbei soweit entwässert, daß die angefallene Schmelze bei 200 - 220° C unter technischen Bedingungen noch rührbar ist, und anschließend die Schmelze unter Ausschluß von Luftsauerstoff auf 250 - 300° C bis zur vollständigen Bildung des Alkalimetallsalzes der Leukoverbindung erhitzt, oder

c) in die Ätzkali-Natriumacetat-Schmelze das Natrium- oder Kaliumsalz des 1,8-Naphthalindicarbonsäureimids bei 200 - 220° C bei Normaldruck einträgt und die Schmelze unter Ausschluß von Luftsauerstoff auf 250 - 300° C bis zur vollständigen Bildung des Alkalimetallsalzes der Leukoverbindung erhitzt.

2. Verfahren nach Anspruch 1 a), dadurch gekennzeichnet, daß man nach Anlegung des Vakuums die Ätzkali-Natriumacetat-Mischung auf 250° bis 300° C erhitzt.

## Claims

1. A process for the preparation of 3,4,9,10-perylenetetracarboxylic acid diimide with a high degree of purity and in a high yield by fusing naphthalene-1,8-dicarboxylic acid imide with potassium hydroxide and sodium acetate to give the alkali metal salt of the leuko compound of 3,4,9,10-perylenetetracarboxylic acid diimide, diluting the melt with water, when the reaction has ended, and oxidizing the leuko form to 3,4,9,10-perylenetetracarboxylic acid diimide, which comprises

a) applying a vacuum of less than 133.3 mbar (100 mm Hg) before the naphthalene-1,8-dicarboxylic acid imide is introduced into the potassium hydroxide/sodium acetate melt, heating the potassium hydroxide/sodium acetate mixture to 200 - 300° C, and hereby dehydrating this mixture such that the melt obtained is still stirrable at 200 - 220° C under industrial conditions, subsequently introducing the naphthalene-1,8-dicarboxylic acid imide or its sodium or potassium salt into the above melt at 200 - 220° C under normal pressure and heating this melt, in the absence of atmospheric oxygen, at 250 - 300° C until formation of the alkali metal salt of the leuko compound is complete, or

b) applying a vacuum of less than 133.3 mbar (100 mm Hg) after the naphthalene-1,8-dicarboxylic acid imide or its sodium or potassium salt has been introduced into the potassium hydroxide/sodium acetate melt at 200 - 220° C under normal pressure, heating the mixture to 200 - 250° C and hereby dehydrating it such that the melt obtained is still stirrable at 200 - 220° C under industrial conditions, and subsequently heating the melt, in the absence of atmospheric oxygen, at 250 - 300° C until formation of the alkali metal salt of the leuko compound is complete, or

c) introducing the sodium or potassium salt of the naphthalene-1,8-dicarboxylic acid imide into the potassium hydroxide/sodium acetate melt at 200 - 220° C under normal pressure and heating the melt, in the absence of atmospheric oxygen, at 250 - 300° C until the formation of the alkali metal salt of the leuko compound is complete.

2. A process as claimed in claim 1, wherein the potassium hydroxide/sodium acetate mixture is heated to 250° C to 300° C after the vacuum has been applied.

## Revendications

1. Procédé pour préparer en un rendement élevé le diimide de l'acide pérylène-tétracarboxylique-3,4,9,10 de degré élevé de pureté, par fusion de l'imide d'acide naphtalène dicarboxylique-1,8 avec de potasse caustique et de l'acétate de sodium pour obtenir le sel de métal alcalin du leuco-dérivé du diimide de l'acide pérylène-tétracarboxylique-3,4,9,10, dilution à l'eau de la masse fondue, après achèvement de la réaction, et oxydation du leuco-dérivé pour obtenir le diimide de l'acide pérylène-tétracarboxylique-3,4,9,10, procédé, caractérisé en ce que

(a) avant d'introduire l'imide de l'acide naphtalène dicarboxylique-1,8 dans la masse fondue potasse caustique/acétate de sodium, on applique un vide inférieur à 133 mbars (100 torrs), on chauffe le mélange potasse caustique/acétate de sodium à 200 - 300° C, et l'on élimine l'eau de ce mélange de manière que la masse fondue obtenue à 200 - 220° C puisse encore être remuée dans des conditions techniques ou industrielles, puis l'on introduit dans la masse fondue citée l'imide d'acide naphtalène dicarboxylique-1,8 ou son sel de sodium ou de potassium, à 200 - 220° C sous la pression normale, et l'on chauffe ensuite cette masse fondue, à l'abri de l'oxygène de l'air, à 250 - 300° C jusqu'à formation complète du sel de métal alcalin du leuco-dérivé, ou

(b) après l'introduction de l'imide d'acide naphtalène dicarboxylique-1,8 ou de son sel de sodium ou de potassium à 200 - 220° C sous la pression normale dans la masse fondue potasse caustique/acétate de sodium, on applique un vide inférieur à 133,3 mbars (100 torrs), on chauffe le mélange à 200 - 250° C et on élimine l'eau de ce mélange de manière que la masse fondue obtenue à 200 - 220° C puisse encore être agitée dans les conditions techniques, et l'on chauffe ensuite, à l'abri de l'oxygène de l'air, la masse fondue à 250 - 300° C jusqu'à formation complète du sel de métal alcalin du leuco-dérivé ou

(c) dans la masse fondue potasse caustique/acétate de sodium, on introduit sous la pression normale, à 200 - 220° C, le sel de sodium ou de potassium de l'imide de l'acide naphtalène dicarboxylique-1,8, et l'on chauffe à l'abri de l'oxygène de l'air la masse fondue jusqu'à 250 - 300° C, jusqu'à formation complète du sel de métal alcalin du leuco-dérivé.

2. Procédé selon la revendication 1(a), caractérisé en ce qu'après application du vide, on chauffe à 250° jusqu'à 300° C le mélange potasse caustique/acétate de sodium.